(19) Europäisches Patentamt
European Patent Office
Office européen des brevets

(11) **EP 4 586 275 A2**

(12) **EUROPEAN PATENT APPLICATION**

(43) Date of publication:
**16.07.2025  Bulletin 2025/29**

(51) International Patent Classification (IPC):
**G16H 50/30** (2018.01)

(21) Application number: **25179999.5**

(22) Date of filing: **16.06.2023**

(52) Cooperative Patent Classification (CPC):
**A61B 5/0205; A61B 5/14557; A61B 5/7275;**
**A61M 1/3609; A61M 1/3666; G16H 20/40;**
**G16H 40/63; G16H 50/30;** A61B 2505/05;
A61M 2230/20; A61M 2230/207; A61M 2230/30

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **17.06.2022  IT 202200012893**

(62) Document number(s) of the earlier application(s) in
accordance with Art. 76 EPC:
**23738580.2 / 4 539 734**

(71) Applicant: **Policlinico San Donato S.p.A.**
**20097 San Donato Milanese (MI) (IT)**

(72) Inventor: **RANUCCI, Marco**
**San Donato Milanese (MI) (IT)**

(74) Representative: **De Gregori, Antonella et al**
**BIRD & BIRD SOCIETA TRA AVVOCATI S.R.L.**
**Via Porlezza, 12**
**20123 Milano (IT)**

Remarks:
This application was filed on 30.05.2025 as a
divisional application to the application mentioned
under INID code 62.

(54) **METHOD AND APPARATUS FOR MONITORING PARAMETERS OF A PATIENT DURING SURGERY WITH EXTRACORPOREAL CIRCULATION**

(57)    The method for monitoring parameters of a patient during surgery with extracorporeal circulation serves to estimate the presence of AKI risk and comprises calculating a dynamic global index of AKI risk as a logistic regression deriving from a static AKI risk index and a dynamic index of AKI risk. The value and/or the time trend of such global index of AKI risk can be advantageously displayed on a monitor during the surgery. The dynamic AKI risk index is calculated at least on the basis of the extracorporeal circulation time, the minimum level of oxygen supply and the exposure time to oxygen supply below a critical threshold which are measured or determined repeatedly during surgery. Preferably, the dynamic AKI risk index is also calculated on the basis of the minimum mean arterial pressure during surgery and/or the maximum concentration of lactate in the blood during surgery and/or the minimum hematocrit during surgery and/or the fact that the patient has been subjected to transfusion(s) during the extracorporeal circulation.

100 — Receive settings of first parameters relating to the patient before starting extracorporeal circulation

200 — Calculate a first static index (SR) of the presence of AKI risk deriving from the first parameters

300 — Receive at least three second parameters (TCPB, DO2, TDO2) relating to the patient during extracorporeal circulation

400 — Calculate at least three second dynamic indices (AKIriskTCPB, AKIriskDO2, AKIriskTDO2) of the presence of AKI risk referred to one of the at least three second parameters, respectively

500 — Calculate a third dynamic index (PRR) of the presence of AKI risk referred to all second indices

600 — Calculate a fourth global index (MDPI) of the presence of AKI risk from the first index (SR) and from the third index (PRR)

700 — Display on a screen a numerical value and/or a time trend of said fourth index (MDPI) during extracorporeal circulation

Fig. 1

EP 4 586 275 A2

## Description

### FIELD OF THE INVENTION

The present invention relates to a method for monitoring parameters of a patient and calculating risk indices deriving from such parameters during surgery with extracorporeal circulation. Such a method may be implemented by a monitoring apparatus adapted to display in particular one or more risk indices.

### BACKGROUND OF THE INVENTION

Extracorporeal circulation, or CardioPulmonary Bypass (=CPB), is an intraoperative technique, i.e. performed during surgeries, used in cardiac surgery with the aim of temporarily replacing the natural function of the heart and lungs of the patient with an artificial function realized by using a heart-lung machine. This technique is typically used to perform so-called "open-heart" surgeries.

Given the non-physiological nature of the extracorporeal circulation, it can generate a number of possible post-operative complications, linked for example to an inflammatory reaction, the need for complete anticoagulation, haemodilution, non-pulsatile flow, etc.

Generally, after complex surgeries and with prolonged extracorporeal circulation, it is not uncommon for organ damage to occur. In particular, apart from cardiac complications, statuses of hepatic insufficiency, impaired lung function, hypoperfusion of the gastrointestinal tract, focal or diffuse brain damage and, above all, renal insufficiency are often reported. In fact, among all the organs, the kidney is one of the most susceptible to the consequences of cardiac surgery and extracorporeal circulation and is also the organ that can provide quantitative indications such as to be able to assess the presence and possible extent of the damage. In this sense, it can be considered a "spy" organ of the multi-organ consequences of cardiac surgery and extracorporeal circulation.

It is in fact known that after surgery, in particular cardiac surgery, or more generally during critical situations to which the patient is subjected, one can witness the onset of Acute Kidney Injury (AKI), which is a clinical syndrome characterized by a rapid reduction in renal function which can be evidenced by the measurement of creatinine.

At present, there are several predictive models of the "static" AKI risk, i.e. based on characteristics of the patient and the surgery that can be defined and quantified before the surgery itself; in other words, this risk is a preoperative AKI risk.

However, other parameters may affect AKI during surgery with extracorporeal circulation; consequently, these parameters, linked to the execution of extracorporeal circulation and therefore of a substantially dynamic type, are not taken into account by predictive "static" risk models. In addition, these models do not allow the possibility of monitoring in real time the trend of the risk, useful to a user, in particular a cardiac anaesthetist and/or a person in charge of the use and management of the heart-lung machine during extracorporeal circulation (i.e. a perfusion technician), in order to be able to modify it with appropriate strategies during extracorporeal circulation itself.

### SUMMARY

The general object of the present invention is to provide a monitoring method that improves the solutions already known.

This general object as well as these and other more specific objects are achieved thanks to what is expressed in the appended claims which form an integral part of the present description.

### LIST OF FIGURES

The present invention will become more readily apparent from the detailed description that follows to be considered together with the accompanying drawings in which:

Fig. 1 shows a block diagram of an embodiment of the method according to the present invention,
Fig. 2 shows an example of graph of the relationship between AKI risk and the minimum level of oxygen supply (DO2) to the patient,
Fig. 3 shows an example of graph of the relationship between AKI risk and exposure time to oxygen supply below a critical threshold ($T_{DO2}$),
Fig. 4 shows an example of graph of the relationship between AKI risk and extracorporeal circulation time ($T_{CPB}$),

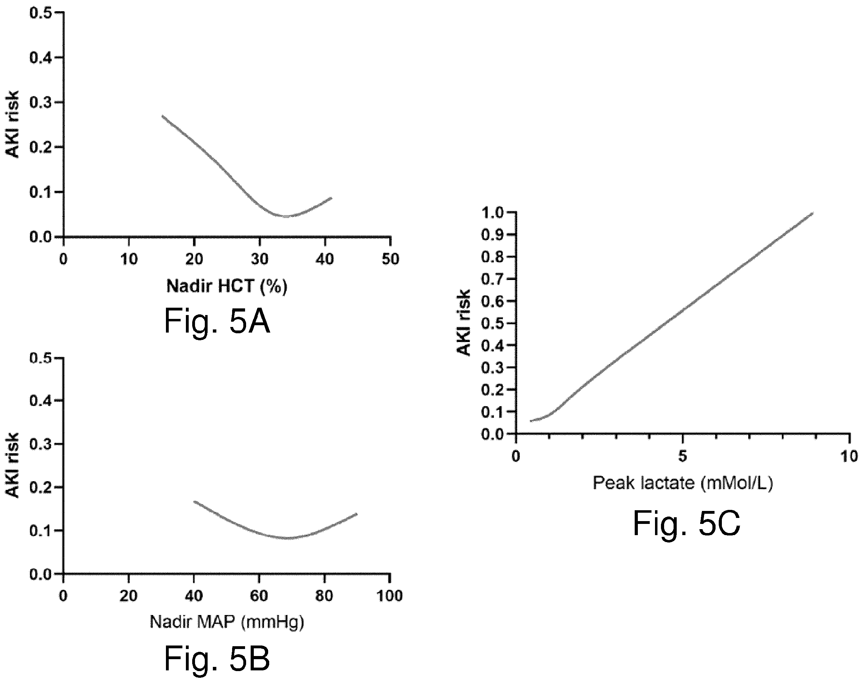

Fig. 5 show three examples of graphs of the relationship between AKI risk and minimum value of hematocrit (HCT), minimum value of mean arterial pressure (MAP) and maximum value of lactates (LAC),
Fig. 6 shows an example of graph representing the ROC (Receiver Operating Characteristic) curves of the first static index (SR), third dynamic index (PRR), and fourth global dynamic index (MDPI), and
Fig. 7 shows an example of displaying on a screen time trends and numerical values according to the present invention.

As can be easily understood, there are various ways of practically implementing the present invention which is defined in its main advantageous aspects in the appended claims and is not limited either to the following detailed description or to the appended claims.

**DETAILED DESCRIPTION**

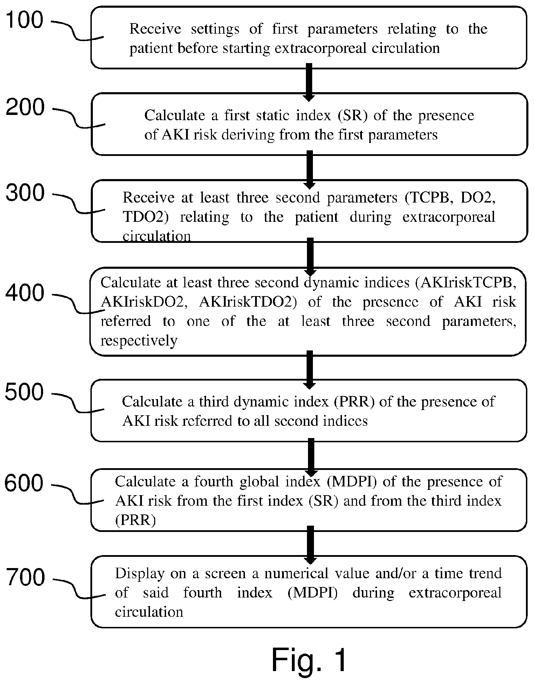

The idea behind the present invention is to provide a method for monitoring parameters of a patient and calculating AKI risk indices deriving from such parameters during surgery with extracorporeal circulation, in particular in the context of cardiac surgery. In particular the present invention proposes both a dynamic AKI risk index and a global AKI risk index which depends on both the dynamic AKI risk index and on a static AKI risk index (i.e. before starting surgery, in particular before starting the extracorporeal circulation) - a static AKI risk index was already known before the present invention. Such dynamic AKI risk index and/or such global AKI risk index is displayed in real time (as a numerical value and/or as a time trend) on a screen of an apparatus during surgery in such a way that a user, in particular a perfusionist technician or a surgeon or a cardiac anaesthetist, can advantageously understand and/or display the trend of such index(ces). Advantageously, the parameters on which this index(ces) depend(s) are also displayed, in such a way that it can be further understood and/or displayed which parameter(s) are modifying this index(ces) the most.
In this way, advantageously, it is also possible to act promptly on such a parameter or such parameters in order to for example minimize the dynamic AKI risk value. In addition, it is important to highlight that the calculated global AKI risk provides an index of the perfusion quality already during the surgical operation.
The global index is not the simple sum of the static index and of the dynamic index; it is instead given by a logistic regression deriving from the static index and from the dynamic index.
The dynamic AKI risk index is calculated at least on the basis of the extracorporeal circulation time, the minimum level of oxygen supply and the exposure time to oxygen supply lower than a critical threshold; these parameters are measured or received repeatedly during extracorporeal circulation.
Preferably, the dynamic AKI risk index is also calculated on the basis of the minimum mean arterial pressure during extracorporeal circulation and/or the maximum concentration of lactates in the blood during extracorporeal circulation and/or the minimum hematocrit during extracorporeal circulation and/or the fact that the patient has been subjected to transfusion(s) during extracorporeal circulation.
The method according to the present invention derives from a study carried out retrospectively on 830 patients operated on in the Cardiac Surgery department of the IRCCS Policlinico San Donato and has been validated by statistical analysis, in particular by ROC (Receiver Operating Characteristic) analysis, also making a comparison with the methods known at present, as will be better explained below.
Typically, a cardiac surgery with extracorporeal circulation involves using a heart-lung machine that temporarily replaces the natural function of the heart and lungs and helps maintain both the supply (perfusion) of blood of the patient, and to provide the oxygen supply necessary for the patient's survival. However, the prolonged exposure of the patient to a low quality of perfusion can lead to unwanted post-operative complications, particularly the risk of renal failure. As known and as already reminded above, the risk of renal failure is assessed using the AKI (Acute Kidney Injury) risk and in particular is called CSA-AKI (Cardiac Surgery Associated Acute Kidney Injury) when it occurs as a post-operative complication after cardiac surgery. It is therefore desirable to have as accurate an estimate as possible of the AKI risk of a patient undergoing surgery with extracorporeal circulation during the surgery itself, so as to have the possibility of monitoring and modifying it (in particular by modifying one or more parameters on which it depends) with appropriate strategies.
As already mentioned, the method according to the present invention serves to monitor parameters of a patient during surgery with extracorporeal circulation to estimate a presence of AKI risk. More particularly, the method according to the present invention serves to display on a screen a numerical value and/or a time trend of at least one index of the presence of AKI risk of a patient during extracorporeal circulation, said index being a global dynamic index of the presence of AKI risk. For example, the method may be carried out by a monitoring apparatus comprising a screen, an electronic computer and an electronic computer program, wherein the electronic computer is connectable to one or more sensors for receiving parameters of a patient and wherein the electronic computer program is adapted to carry out the method according to the present invention when executed by the electronic computer. With non-limiting reference to Fig. 1, the method generally comprises the following steps:

A. receiving 100 from devices and/or apparatuses first parameters relating to the patient before starting extracorporeal circulation;

B. calculating 200 a first index SR deriving from the first parameters received in step A, the first index SR being a static index of the presence of AKI risk;

C. receiving 300 from devices and/or apparatuses at least three second parameters $T_{CPB}$, DO2, $T_{DO2}$ relating to the patient during extracorporeal circulation, wherein the at least three second parameters are an extracorporeal circulation time $T_{CPB}$, a minimum level of oxygen supply DO2 and an exposure time $T_{DO2}$ to oxygen supply lower than (below) a critical threshold, and wherein the at least three second parameters are received and updated during extracorporeal circulation;

D. calculating 400 at least three second indices AKIriskT$_{CPB}$, AKIriskDO2 and AKIriskT$_{DO2}$ during extracorporeal circulation, each of the at least three second indices AKIriskT$_{CPB}$, AKIriskDO2 and AKIriskT$_{DO2}$ being deriving respectively from at least one of the at least three second parameters $T_{CPB}$, DO2 and $T_{DO2}$ received in step C, each of the at least three second indices AKIriskT$_{CPB}$, AKIriskDO2 and AKIriskT$_{DO2}$ being dynamic indices of the presence of AKI risk respectively referred to one of said at least three second parameters $T_{CPB}$, DO2 and $T_{DO2}$;

E. calculating 500 a third index PRR during extracorporeal circulation by means of a logistic regression deriving from the at least three second indices calculated in step D, wherein the third index PRR is a dynamic index of the presence of AKI risk referred to all second indices calculated in step D;

F. calculating 600 a fourth index MDPI during extracorporeal circulation by means of a logistic regression deriving from the first index SR and from the third index PRR, wherein the fourth index MDPI is a global dynamic index of the presence of AKI risk;

G. displaying 700 on a screen a numerical value 710 and/or a time trend 720 of the fourth index MDPI during extracorporeal circulation.

It should be noted that in step G the time trend displayed on the screen is advantageously a graphical display (for example by means of a line graph) of the variation of the fourth index MDPI over time.

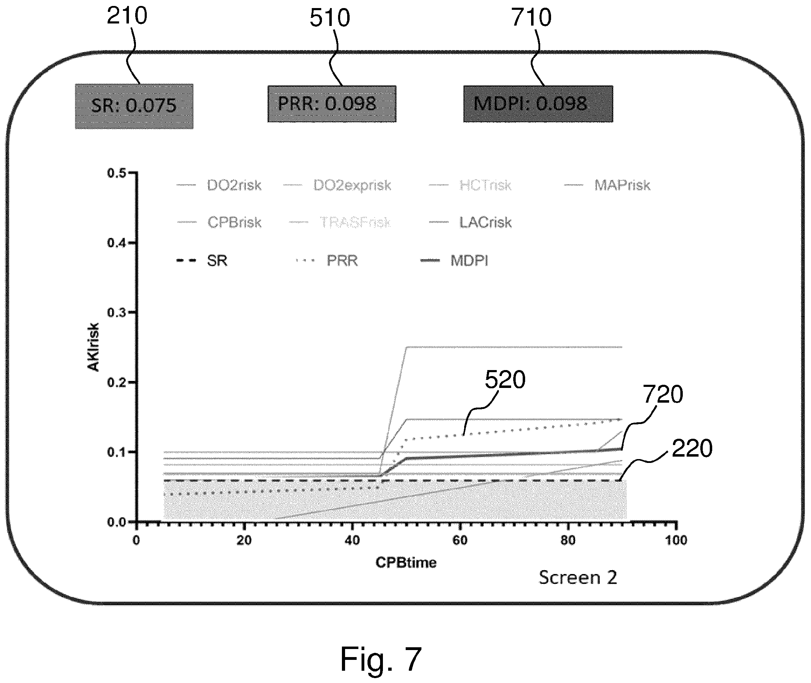

As will be better understood when describing Fig. 7, according to this example the received second parameters and the calculated second indices are advantageously greater than three in number; more precisely, they are respectively seven parameters and seven indices, from which the calculation of the third dynamic index PRR of the presence of the AKI risk referred to all seven of the calculated second indices derives. However, the method according to the present invention may be implemented by receiving at least three second parameters and consequently at least three second indices, each deriving respectively from at least one of the three previously received second parameters. Below reference will be made to two non-limiting examples of implementation of the method: a first example in which only three second parameters and a second example are received, in particular more advantageous (having greater sensitivity), in which seven second parameters are received.

The three second parameters that at least must be received are:

- the extracorporeal circulation time $T_{CPB}$, i.e. the time in which the patient is subjected to extracorporeal circulation;
- the minimum level of oxygen supply DO2, or the level of oxygen supplied to the patient, in particular by means of the heart-lung machine, this level being a minimum level;
- the time $T_{DO2}$ of exposure to oxygen supply lower than a critical threshold, i.e. the time in which the patient is subjected to a critical level of oxygen supply (where the criticality is due in particular to a low oxygen supply, i.e. an oxygen supply lower than a critical threshold), in particular by means of the heart-lung machine.

As mentioned, the calculation of the fourth index (MDPI = Multifactorial Dynamic Perfusion Index) derives from a first index (SR = Static Risk) and from a third index (PRR = Perfusion Related Risk), respectively a static index and a dynamic index of the presence of AKI risk. The first static index SR is calculated from first parameters relating to the patient before starting extracorporeal circulation. In fact, it is known that for a patient who must undergo cardiac surgery, based on some parameters of the patient himself and some parameters of the surgery itself, it can be calculated a static predictive risk index of renal failure. A previous study identified the so-called Cleveland Clinic Score (=CCS), a clinical score to predict acute kidney damage (AKI risk) after cardiac surgery that incorporates the effect of its main risk factors, such as the sex of the patient, the type of surgery, the possible emergency of the surgery, the value of baseline creatinine, the ejection fraction of the patient, the presence of a chronic obstructive pulmonary disease, the presence of diabetes, the execution of a previous cardiac surgery and the use of an aortic counterpulsor. These parameters are typically collected and set by a user, for example by means of a keyboard or touchscreen, before performing cardiac surgery, in particular before starting extracorporeal circulation. The static risk index SR according to the present invention was developed starting from the already known Cleveland Clinic Score. However, unlike the Cleveland Clinic Score, it does not include the baseline creatinine value among the first parameters, as there is a mathematical link between the baseline creatinine value and the creatinine value that defines the AKI risk. Furthermore, the static risk index SR calculated according to the present invention further includes the parameter "age" and the parameter "HCT", i.e. the preoperative hematocrit value of the blood (i.e. the percentage of blood volume occupied by red blood cells). In general, these parameters are also set or received before starting cardiac surgery, in particular before starting extracorporeal circulation. It should be noted that one or more of the first parameters from which the first index SR is derived may be received and/or set manually by a user or they may be received and/or set automatically. In general, these first parameters are received by devices and/or apparatuses, in particular devices and/or apparatuses of an electronic computer. For example, the devices may be a keyboard and/or a mouse and/or a microphone and/or a touchscreen (i.e. components of an electronic computer) and the apparatuses may be a memory disk and/or a communication card (i.e. components of an electronic computer). Advantageously, also the screen on which a numerical value and/or a time trend of the fourth index MDPI is/are displayed (and possibly also of the first index SR and/or of the third index PRR, as will be better described below) is a component of an electronic computer. Advantageously, the first static index SR of the presence of AKI risk calculated in step B of the method is calculated by the following formula, in particular according to the logistic equation:

$$SR = exp\ (-1.017 + A1 * CCS + A2 * E + A3 * HCT)/[1 + exp\ (-1.017 + A1 * CCS + A2 * E + A3 * HCT)]$$

wherein:

- CCS is the Cleveland Clinic Score,
- E it is the patient's age measured in years [years],
- HCT is the percentage of blood volume occupied by red blood cells (hematocrit) measured in percentage [%],
- A1 is a numerical value comprised between -0.2 and 0.7, preferably comprised between 0 and 0.27, more preferably equal to 0.078,
- A2 is a numerical value comprised between 0.011 and 0.06, preferably comprised between 0.015 and 0.055, more preferably equal to 0.035, and
- A3 is a numerical value comprised between -0.17 and 0.015, preferably comprised between
- 0.14 and -0.05, more preferably equal to -0.095.

In particular, the first static index SR of the presence of AKI risk can assume a value between 0 (corresponding to the absence of risk) and 1 (corresponding to 100% risk). Note that this first index SR does not vary over time as it does not depend on quantities that vary over time. As a result, the calculated index SR is a constant value. Advantageously, as shown for example in Fig. 7, the method also includes a step B0 of displaying on a screen a numerical value 210 and/or a constant stroke 220 (dashed line) of the index SR.

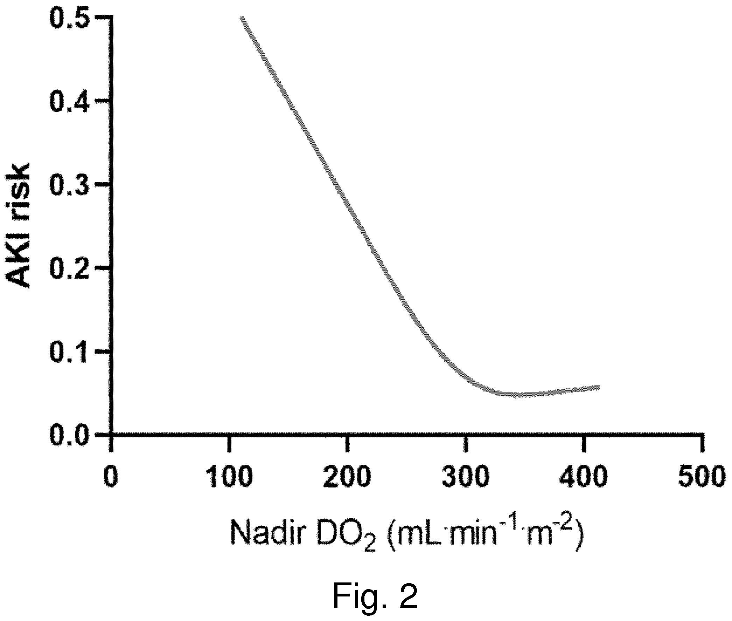

As already mentioned, step C of the method comprises receiving 300 at least three second parameters and step D of calculating 400 at least three second indices, each deriving respectively from at least one of the three second parameters and referred respectively to one of the three second parameters. With non-limiting reference to Figs. 2-4 and according to a first embodiment example, the three second parameters received in step C are the extracorporeal circulation time $T_{CPB}$, the minimum level of oxygen supply DO2 and the exposure time $T_{DO2}$ to oxygen supply below a critical threshold. For example, such parameters may be calculated automatically by means of sensors and/or timers connected to the monitoring apparatus. For each of the second parameters received in step C, a second dynamic risk of the presence of AKI risk is then calculated in step D. In other words, for each of the second parameters received, the association of the parameter with the AKI risk was studied and verified and in particular the equation that determines the relationship between the parameter and the AKI risk was calculated. With reference to Fig. 2, the graphic relationship between the minimum level of oxygen supply DO2 and the AKI risk associated therewith is shown, i.e. the second oxygen supply-related index AKIriskDO2 is shown graphically. Advantageously, said second index AKIriskDO2 is calculated by the formula:

- linear regression within a first predetermined range of values of the minimum level of oxygen supply DO2, in particular between 0 and 300 [mL/min/m$^2$], preferably between 0 and 289 [mL/min/m$^2$]:

$$AKIriskDO2\ =\ B3 + B4 * DO2$$

wherein:

- B3 is a numerical value comprised between 0.25 and 1.09, preferably comprised between 0.35 and 0.98, more preferably equal to 0.67,
- B4 is a numerical value comprised between -0.005 and -0.0006, preferably comprised between -0.003 and -0.001, more preferably equal to -0.002;
- constant within a second range of values, in particular between 289 and 800 [mL/min/m$^2$], wherein the constant is in particular equal to 0.053 (i.e. a percentage of the presence of constant AKI risk and equal to 5.3%);

wherein DO2 is a quantity measured in [mL/min/m$^2$].

Note that the range of values between 262 [mL/min/m$^2$] and 300 [mL/min/m$^2$] is considered to contain a critical DO2 threshold; preferably, the critical DO2 threshold is 289 [mL/min/m$^2$]. It is important to highlight that DO2 is the minimum level of oxygen supply reached and maintained for at least 5 minutes. In other words, subsequent decreases in DO2 with a duration of less than 5 minutes do not modify the DO2 variable of the formula, as do subsequent increases in DO2 (remember that DO2 is a minimum value).

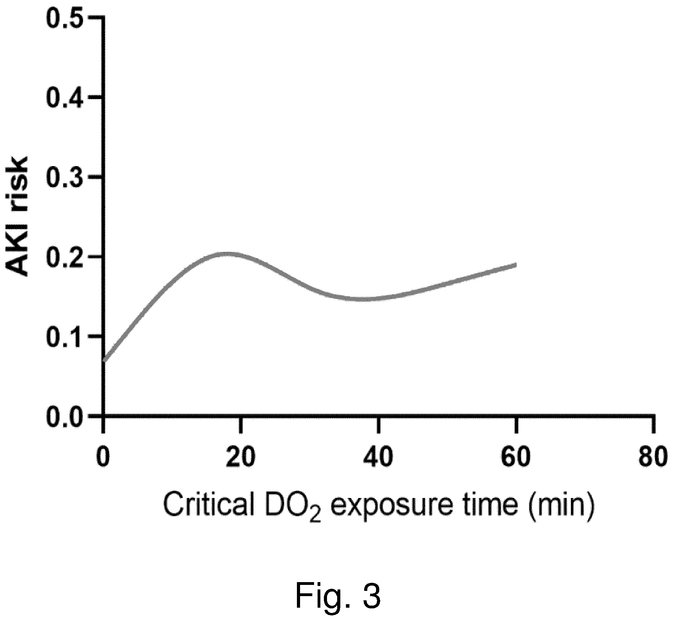

With reference to Fig. 3, the graphic relationship between the time $T_{DO2}$ of exposure to oxygen supply below a critical threshold and the AKI risk associated therewith is shown, i.e. the second index $AKIriskT_{DO2}$ relating to the time of exposure to oxygen supply below a critical threshold is graphically shown. In particular, the critical threshold of oxygen supply is comprised in the range of values between 262 [mL/min/m$^2$] and 300 [mL/min/m$^2$], preferably, the critical threshold is 289 [mL/min/m$^2$].

Advantageously, this second index $AKIriskT_{DO2}$ is calculated by the formula:

- cubic regression within a first predetermined range of values of exposure time $T_{DO2}$ to oxygen supply below a critical threshold, in particular between 2 and 360 [min]:

$$AKIriskT_{DO2} = B5 + B6 * T_{DO2} + B7 * (T_{DO2})^2 + B8 * (T_{DO2})^3$$

wherein:

- B5 is a numerical value comprised between 0.036 and 0.10, preferably comprised between 0.044 and 0.092, more preferably equal to 0.068,
- B6 is a numerical value comprised between 0.002 and 0.03, preferably comprised between 0.0052 and 0.0261, more preferably equal to 0.016,
- B7 is a numerical value comprised between -0.0014 and 0.00027, preferably comprised between -0.0012 and 0.000066, more preferably equal to -0.000557,
- B8 is a numerical value comprised between -0.00000691 and 0.0000153, preferably comprised between -0.00000388 and 0.0000153, more preferably equal to 0.00000183;
- constant within a second predetermined range of values, in particular between 0 and 2 [min], the constant value being in particular equal to 0.05 (i.e. a percentage of the presence of constant AKI risk and equal to 5%);

wherein $T_{DO2}$ is a quantity measured in [min].

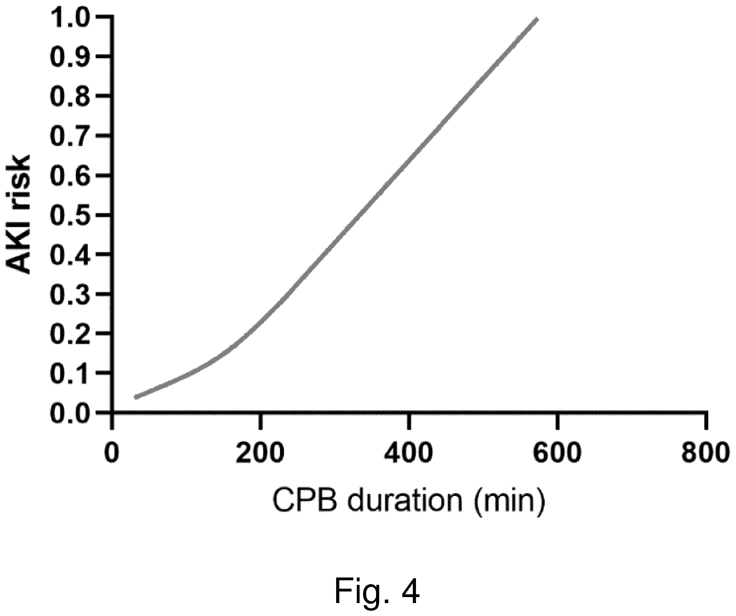

With reference to Fig. 4, the graphic relationship between the extracorporeal circulation time $T_{CPB}$ and the AKI risk associated therewith is shown, i.e. the second index $AKIriskT_{CPB}$ relating to the extracorporeal circulation time is graphically shown.

Advantageously, this second index $AKIriskT_{CPB}$ is calculated by the formula: linear regression of the extracorporeal circulation time $T_{CPB}$ within a predetermined range of values of extracorporeal circulation time $T_{CPB}$, in particular between 30 and 360 [min]:

$$AKIriskT_{CPB} = B1 + B2 * T_{CPB}$$

wherein:

- B1 is a numerical value comprised between -0.18 and 0.027, preferably comprised between
- 0.074 and 0.015, more preferably equal to -0.029,
- B2 is a numerical value comprised between 0.0008 and 0.0048, preferably comprised between 0.00092 and 0.00172, more preferably equal to 0.0013;

wherein $T_{CPB}$ is a quantity measured in [min].

It should be noted that, according to some values of $T_{CPB}$, the value of the second parameter $AKIriskT_{CPB}$ could be less than zero: in this situation, or if $AKIriskT_{CPB} < 0$, a zero risk is assumed (i.e. $AKIriskT_{CPB} = 0$).

Advantageously, the method also includes a step D0 of displaying on a screen a numerical value and/or a time trend of one or more of the at least three second indices $AKIriskT_{CPB}$, AKIriskDO2, $AKIriskT_{DO2}$ calculated in step D (as shown for example in Fig. 7). In particular, the time trend is referred to the extracorporeal circulation time $T_{CPB}$, i.e. the variation of one or more of the at least three second indices $AKIriskT_{CPB}$, AKIriskDO2, $AKIriskT_{DO2}$ as the extracorporeal circulation time $T_{CPB}$ varies is graphically displayed.

The method further provides, in step E, for the calculation of the third index PRR during extracorporeal circulation by means of a logistic regression deriving from the at least three second indices calculated in step D, wherein the third index PRR is a dynamic index of the presence of AKI risk referred to all the second indices calculated in step D. Advantageously, in the present embodiment, in step D the three indices $AKIriskT_{CPB}$, AKIriskD02, $AKIrisk_{DO2}$ have been calculated and the third index PRR calculated in step E is calculated by the formula:

$PRR = exp(C1 + C2 * AKIriskT_{CPB} + C4 * AKIriskDO2 + C6 * AKIriskT_{DO2})/[1 + exp(C1 + C2 * AKIriskT_{CPB} + C4 * AKIriskDO2 + C6 * AKIriskT_{DO2})]$

wherein:

- C1 is a numerical value comprised between -6.4 and -1.82, preferably comprised between - 5.83 and -2.39, more preferably equal to -4.11,
- C2 is a numerical value comprised between -0.01 and 9.2, preferably comprised between 1.03 and 8.07, more preferably equal to 4.55,
- C4 is a numerical value comprised between -3.7 and 11.3, preferably comprised between - 1.88 and 9.48, more preferably equal to 3.8, and
- C6 is a numerical value comprised between -9.3 and 9.8, preferably comprised between - 6.94 and 7.5, more preferably equal to 0.28.

However, as already mentioned and as will be better explained later, in step C several second parameters, in particular 7 second parameters, can be advantageously received and in step D several second indices, in particular 7 second indices, can be calculated.

Advantageously, as shown for example in Fig. 7, the method also includes a step E0 of displaying on a screen a numerical value 510 and/or a time trend 520 (dotted line) of the third index PRR calculated in step E. In particular, the time trend 520 refers to the extracorporeal circulation time $T_{CPB}$, i.e. the variation of the third index PRR as the extracorporeal circulation time $T_{CPB}$ varies is displayed graphically.

Finally, the method provides a step F of calculating a fourth index MDPI during extracorporeal circulation by means of a logistic regression deriving from the first index SR and from the third index PRR, wherein the fourth index MDPI is a global dynamic index of the presence of AKI risk, and a step G of displaying on a screen during extracorporeal circulation a numerical value 710 and/or a time trend 720 of the fourth index MDPI calculated in step F. In particular, the fourth index calculated in step F is calculated by the formula:

$$MDPI = exp(D1 + D2 * SR + D3 * PRR)/[1 + exp(D1 + D2 * SR + D3 * PRR)]$$

wherein:

- D1 is a numerical value comprised between -3.95 and -2.74, preferably comprised between
- 3.8 and -2.9, more preferably equal to -3.35,
- D2 is a numerical value comprised between 0.33 and 8.4, preferably comprised between 1.25 and 7.09, more preferably equal to 4.17,
- D3 is a numerical value comprised between 2.58 and 10.15, preferably comprised between 3.31 and 7.41, more preferably equal to 5.37.

Finally, with non-limiting reference to Fig. 7, an example of displaying on a screen the numerical value 710 and the time trend 720 of the fourth index MDPI is shown, wherein the time trend refers to the extracorporeal circulation time $T_{CPB}$, i.e. the variation of the fourth index MDPI as the extracorporeal circulation time $T_{CPB}$ varies is graphically displayed.

Advantageously, the method further comprises a step G0 of comparing the fourth index MDPI with respect to the first index SR and activating signalling means as a function of the difference between the fourth index MDPI and the first index SR; in particular, the signalling means may be a graphic signalling, for example the display of an alarm message, and/or an audible signalling, for example the reproduction of an alarm sound; in particular such signalling or such signallings may occur if the difference between MDPI and SR is greater than a predetermined value (e.g. configurable) or a variable value depending for example on the value SR (for example a signalling could occur if MDPI is greater than SR by 50%); in particular, such signalling or such signallings may occur only if a certain condition lasts for at least a predetermined time; in particular, differentiated signallings may be envisaged depending on the severity of the situation.

As already mentioned, a second non-limiting embodiment example will be described below in which in step C seven second parameters are received and in step D seven second indices referred respectively to each of the parameters received in step C are calculated. Note thin step A, step B, step F and step G of the method do not differ between these embodiment examples. In other words, only step C, step D and step E have differences between the first embodiment example and the second embodiment example.

In particular, in addition to the three aforementioned parameters (i.e. extracorporeal circulation time $T_{CPB}$, minimum level of oxygen supply DO2, and exposure time $T_{DO2}$ to oxygen supply below a critical threshold), in step C it is further and advantageously received as follows:

- a minimum value of mean arterial pressure MAP measured in [mmHg], wherein MAP is equal to the minimum value of mean arterial pressure detected during the extracorporeal circulation time $T_{CPB}$ and wherein the detection is an automatic detection;
- a maximum value of blood lactate concentration LAC in the blood detected during the extracorporeal circulation time $T_{CPB}$ and measured in [mMol/L], wherein the detection is a manual detection;
- a minimum hematocrit value HCT detected during the extracorporeal circulation time $T_{CPB}$ and measured in [%], wherein the detection is an automatic detection; and
- transfusion information TRANSF detected during the extracorporeal circulation time $T_{CPB}$, wherein the detection is a manual detection.

Accordingly, step D of the method according to this second embodiment further comprises calculating:

- a second index AKIriskMAP deriving from the mean minimum blood pressure MAP value and calculated by the formula:

  ■ quadratic regression, in particular

$$AKIriskMAP = B9 + B10 * MAP + B11 * (MAP)^2$$

  wherein:

  • B9 is a numerical value comprised between -0.43 and 1.71, preferably comprised between - 0.17 and 1.45, more preferably equal to 0.64,
  • B10 is a numerical value comprised between -0.05 and 0.0188, preferably comprised between -0.042 and 0.0098, more preferably equal to -0.0162,
  • B11 is a numerical value comprised between -0.000152 and 0.00039, preferably comprised between -0.0000871 and 0.000323, more preferably equal to 0.00012;

- a second index AKIriskLAC, deriving from the maximum value of lactates LAC and calculated by the formula:

  ■ linear regression, in particular

$$AKIriskLAC = B12 + B13 * LAC$$

  wherein:

  • B12 is a numerical value comprised between -0.1 and 0.32, preferably comprised between - 0.06 and 0.20, more preferably equal to -0.02,
  • B13 is a numerical value comprised between 0.069 and 0.155, preferably comprised between 0.08 and 0.145, more preferably equal to 0.11;

- a second index AKIriskHCT deriving from the minimum hematocrit value HCT and calculated by the formula:

  ■ linear regression within a first predetermined range of values of minimum hematocrit (HCT), in particular between 10 and 26 [%]

$$AKIriskHCT = B14 + B15 * HCT$$

  wherein:

- B14 is a numerical value comprised between -0.16 and 1.9, preferably comprised between 0.144 and 1.15, more preferably equal to 0.65,
- B15 is a numerical value comprised between -0.082 and 0.014, preferably comprised between -0.042 and 0.001, more preferably equal to -0.02;

  ▪ substantially constant within a second predetermined range of values, in particular between 26 and 100 [%], wherein the constant is in particular equal to 0.064 (i.e. a percentage of the presence of AKI risk equal to 6.4%);

- a second index AKIriskTRANSF deriving from the information of said transfusion TRANSF and calculated by the formula:

  ▪ constant if the patient is not subject to transfusion, the constant being in particular equal to 0.082 (i.e. a percentage of the presence of AKI risk equal to 8.2%);
  ▪ constant if the patient is subject to transfusion, the constant being in particular equal to 0.293 (or a percentage of the presence of AKI risk equal to 29.3%).

It is important to note that MAP is the minimum average blood pressure reached and maintained for at least 5 minutes. In other words, subsequent decreases in MAP with a duration of less than 5 minutes do not change the variable MAP of the formula, as do successive increases in MAP (remember that MAP is a minimum value). Similarly, HCT is the minimum hematocrit value reached and maintained for at least 5 minutes. In other words, subsequent decreases in HCT with a duration of less than 5 minutes do not change the HCT variable of the formula, subsequent increases in HTC (remember that HTC is a minimum value).

According to this second embodiment, the third index PRR calculated in step E during extracorporeal circulation further derives also from the second indices AKIriskMAP, AKIriskLAC, AKIriskHCT, AKIriskTRANSF and is advantageously calculated by the formula:

$$
\begin{aligned}
PRR = exp(&C1 + C2 * AKIriskT_{CPB} + C3 * AKIriskLAC + C4 * AKIriskDO2 + C5 \\
&* AKIriskHCT + C6 * AKIriskT_{DO2} + C7 * AKIriskTRANSF + C8 \\
&* AKIriskMAP)/[1 + exp(C1 + C2 * AKIriskT_{CPB} + C3 * AKIriskLAC \\
&+ C4 * AKIriskDO2 + C5 * AKIriskHCT + C6 * AKIriskT_{DO2} + C7 \\
&* AKIriskTRANSF + C8 * AKIriskMAP)]
\end{aligned}
$$

wherein:

- C1 is a numerical value comprised between -6.4 and -1.8, preferably comprised between - 5.83 and -2.39, more preferably equal to -4.11,
- C2 is a numerical value comprised between -0.01 and 9.2, preferably comprised between 1.03 and 8.07, more preferably equal to 4.55,
- C3 is a numerical value comprised between 0.07 and 8.33, preferably comprised between 1.07 and 7.33, more preferably equal to 4.2,
- C4 is a numerical value comprised between -3.7 and 11.3, preferably comprised between - 1.88 and 9.48, more preferably equal to 3.8,
- C5 is a numerical value comprised between -7.3 and 8.5, preferably comprised between -5.3 and 6.46, more preferably equal to 0.58,
- C6 is a numerical value comprised between -9.3 and 9.8, preferably comprised between - 6.94 and 7.5, more preferably equal to 0.28,
- C7 is a numerical value comprised between -1.15 and 8.44, preferably comprised between 0.01 and 7.29, more preferably equal to 3.65,
- C8 is a numerical value comprised between -19.8 and 20.8, preferably comprised between - 0.48 and 1.48, more preferably equal to 0.5.

Note that the formula for calculating the third index PRR reported in the first embodiment substantially coincides with the formula just reported in the second embodiment wherein the values of AKIriskLAC, AKIriskHCT, AKIriskTRANS and AKIriskMAP are null, since in step C of the first embodiment example only the three parameters $T_{CPB}$, DO2 and $T_{DO2}$

were received and consequently only the three indices AKIriskT$_{CPB}$, AKIriskDO2 and AKIriskT$_{DO2}$ were calculated.

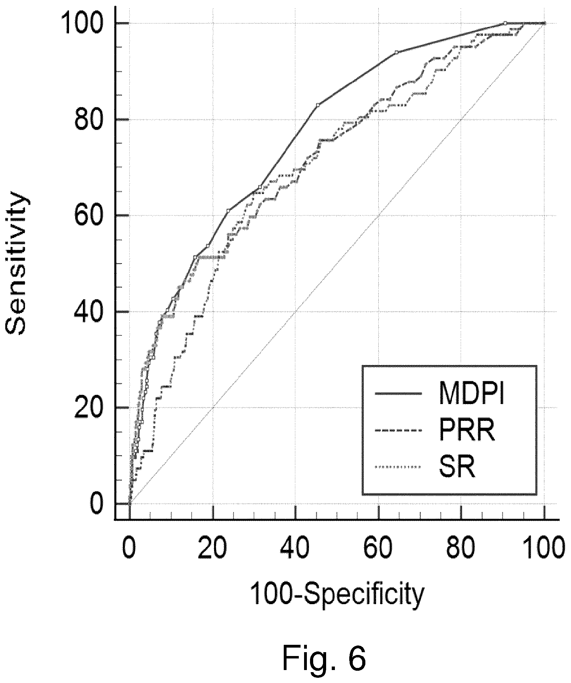

As already mentioned, the method according to the present invention has been validated by statistical analysis, in particular by ROC (=Receiver Operating Characteristic) analysis of the fourth index MDPI and advantageously also of the first index SR and of the third index PRR (see for example Fig. 6). In the first embodiment described, i.e. the case where only the three second parameters T$_{CPB}$, DO2 and T$_{DO2}$ are received in step C (and consequently in step D only three second indices referred respectively to the three second parameters received are calculated), the ROC analysis of the fourth index MDPI yielded an Area Under the Curve (=AUC) equal to 0.75. In the second embodiment described, i.e. the case where in step C seven second parameters are received (and consequently in step D seven second indices referred respectively to the seven second parameters received are calculated), the ROC analysis of the fourth index MDPI (shown in Fig. 6) yielded an Area Under the Curve (=AUC) equal to 0.767.

As known to the persons expert in the sector, the closer the AUC is to 1 the greater is the performance of a model (AUC is therefore an indicator of the discriminatory power of the model). Consequently, although the AUC of the first embodiment was already to be considered a good indicator, the AUC of the second embodiment indicates that this model is more performant. It should also be noted that the graph shown in Fig. 6 also reports the ROC curves of only the first index SR and only of the third index PRR; the Area Under the Curve was calculated for both, which was equal to 0.696 for the first index SR and 0.723 for the third index PRR, respectively. In summary, it can therefore be concluded that the fourth index MDPI integrating the first index SR and the third index PRR has a greater discriminatory power than the individual indices SR and PRR.

**Claims**

1. Method for monitoring parameters of a patient during surgery with extracorporeal circulation to estimate a presence of AKI risk, the method comprising the steps of:

   A. receiving (100) from devices and/or apparatuses first parameters relating to the patient before starting extracorporeal circulation;
   B. calculating (200) a first index (SR) deriving from said first parameters received in step A, said first index (SR) being a static index of the presence of AKI risk;
   C. receiving (300) from devices and/or apparatuses at least three second parameters (T$_{CPB}$, DO2, T$_{DO2}$) relating to the patient during extracorporeal circulation, said at least three second parameters being an extracorporeal circulation time (T$_{CPB}$), a minimum level of oxygen supply (DO2) and an exposure time to oxygen supply below a critical threshold (T$_{DO2}$), wherein said at least three second parameters are received and updated during extracorporeal circulation;
   D. calculating (400) at least three second indices (AKIriskT$_{CPB}$, AKIriskDO2, AKIriskT$_{DO2}$) during extracorporeal circulation, each of said at least three second indices (AKIriskT$_{CPB}$, AKIriskD02, AKIriskT$_{DO2}$) being deriving respectively from at least one of said at least three second parameters (T$_{CPB}$, DO2, T$_{DO2}$) received in step C, each of said at least three second indices (AKIriskT$_{CPB}$, AKIriskDO2, AKIriskT$_{DO2}$) being dynamic indices of the presence of AKI risk respectively referred to one of said at least three second parameters (T$_{CPB}$, DO2, T$_{DO2}$);
   E. calculating (500) a third index (PRR) during extracorporeal circulation by means of a logistic regression deriving from said at least three second indices calculated in step D, said third index (PRR) being a dynamic index of the presence of AKI risk referred to all second indices calculated in step D;
   F. calculating (600) a fourth index (MDPI) during extracorporeal circulation by means of a logistic regression deriving from said first index (SR) and said third index (PRR), said fourth index (MDPI) being a global dynamic index of the presence of AKI risk;
   G. displaying (700) on a screen a numerical value (710) and/or a time trend (720) of said fourth index (MDPI) during extracorporeal circulation.

2. Method according to claim 1, wherein the first parameters received in step A comprise patient's age, percentage of blood volume occupied by red blood cells (hematocrit) and Cleveland Clinic Score,

   wherein said first index (SR) calculated in step B is calculated by the formula:

   $SR = exp(-1.017 + A1 * CCS + A2 * E + A3 * HCT)/[1 + exp(-1.017 + A1 * CCS + A2 * E + A3 * HCT)]$

   wherein:

- CCS is the Cleveland Clinic Score,
- E is the patient's age,
- HCT is the percentage of blood volume occupied by red blood cells (hematocrit),
- A1 is a numerical value comprised between -0.2 and 0.7, preferably comprised between 0 and 0.27, more preferably equal to 0.078,
- A2 is a numerical value comprised between 0.011 and 0.06, preferably comprised between 0.015 and 0.055, more preferably equal to 0.035,
- A3 is a numerical value comprised between -0.17 and 0.015, preferably comprised between -0.14 and -0.05, more preferably equal to -0.095;

wherein E and HCT are quantities measured in [years] and [%], respectively.

3. Method according to claim 1 or 2, wherein one of said at least three second indices is an index relating to the extracorporeal circulation time (AKIriskT$_{CPB}$) and is calculated by the formula:

linear regression of the extracorporeal circulation time (T$_{CPB}$) within a predetermined range of values of extracorporeal circulation time (T$_{CPB}$), in particular between 30 and 360 [min]:

$$AKIriskT_{CPB} \; = \; B1 + B2 * T_{CPB}$$

wherein:

- B1 is a numerical value comprised between -0.18 and 0.027, preferably comprised between -0.074 and 0.015, more preferably equal to -0.029,
- B2 is a numerical value comprised between 0.0008 and 0.0048, preferably comprised between 0.00092 and 0.00172, more preferably equal to 0.0013;

wherein T$_{CPB}$ is a quantity measured in [min];
and wherein if $AKIriskT_{CPB} < 0$ then $AKIriskT_{CPB} = 0$.

4. Method according to claim 1 or 2 or 3, wherein one of said at least three second indices is an index relating to the minimum level of oxygen supply (AKIriskD02) and is calculated by the formula:

- linear regression within a first predetermined range of values of oxygen supply (DO2), in particular between 0 and 289 [mL/min/m$^2$]:

$$AKIriskDO2 \; = \; B3 + B4 * DO2$$

wherein:

- B3 is a numerical value comprised between 0.25 and 1.09, preferably comprised between 0.35 and 0.98, more preferably equal to 0.67,
- B4 is a numerical value comprised between -0.005 and -0.0006, preferably comprised between -0.003 and -0.001, more preferably equal to -0.002;
- constant within a second range of values, in particular between 289 and 800 [mL/min/m$^2$], said constant being in particular equal to 0.053;

wherein DO2 is a quantity measured in [mL/min/m$^2$].

5. Method according to claim 1 or 2 or 3 or 4, wherein one of said at least three second indices is an index relating to the time of exposure to oxygen supply below a critical threshold (AKIriskT$_{DO2}$) and is calculated by the formula:

- cubic regression within a first predetermined range of values of exposure time to oxygen supply below a critical threshold (T$_{DO2}$), in particular between 2 and 360 [min]:

$$AKIriskT_{DO2} \; = \; B5 + B6 * T_{DO2} + B7 * (T_{DO2})^2 + B8 * (T_{DO2})^3$$

wherein:

- B5 is a numerical value comprised between 0.0036 and 0.1, preferably comprised between 0.044 and 0.092, more preferably equal to 0.068,
- B6 is a numerical value comprised between 0.0002 and 0.03, preferably comprised between 0.0052 and 0.0261, more preferably equal to 0.016,
- B7 is a numerical value comprised between -0.0014 and 0.00027, preferably comprised between -0.0012 and 0.000066, more preferably equal to -0.000557,
- B8 is a numerical value comprised between -0.00000691 and 0.0000183, preferably comprised between -0.00000388 and 0.0000153, more preferably equal to 0.00000573;
- constant within a second predetermined range of values, in particular between 0 and 2 [min], said constant being in particular equal to 0.05;

wherein $T_{DO2}$ is a quantity measured in [min],
wherein said critical threshold is comprised in the range of values between 262 to 300 [mL/min/m$^2$], in particular said critical threshold is equal to 289 [mL/min/m$^2$].

6. Method according to any one of the preceding claims, wherein step C further comprises receiving a minimum value of mean arterial pressure (MAP), said value being equal to the minimum value of mean arterial pressure detected during the extracorporeal circulation time ($T_{CPB}$), wherein the mean arterial pressure detection is an automatic detection,

and wherein the calculation step D further comprises calculating a second index deriving from said minimum value of mean arterial pressure (AKIriskMAP) said second index being calculated by the formula:

- quadratic regression, in particular

$$AKIriskMAP = B9 + B10 * MAP + B11 * (MAP)^2$$

wherein:

- B9 is a numerical value comprised between -0.43 and 1.471 preferably comprised between -0.17 and 1.45, more preferably equal to 0.64,
- B10 is a numerical value comprised between -0.05 and 0.018, preferably comprised between -0.042 and 0.0098, more preferably equal to -0.0162,
- B11 is a numerical value comprised between -0.000152 and 0.00039, preferably comprised between -0.0000871 and 0.000323, more preferably equal to 0.00012;

wherein MAP is a quantity measured in [mmHg];
wherein, in step E, said third index (PRR) is calculated during extracorporeal circulation by means of a logistic regression also deriving from said second index.

7. Method according to any one of the preceding claims, wherein step C further comprises receiving a maximum value of blood lactate concentration (LAC) detected during the extracorporeal circulation time ($T_{CPB}$), said detection being a manual detection,

and wherein the calculation step D further comprises calculating a second index deriving from said maximum value of lactate concentration (AKIriskLAC), said second index being calculated by the formula:

- linear regression, in particular

$$AKIriskLAC = B12 + B13 * LAC$$

wherein:

- B12 is a numerical value comprised between -0.1 and 0.032 preferably comprised between -0.06 and 0.20, more preferably equal to -0.02,
- B13 is a numerical value comprised between 0.069 and 0.155, preferably comprised between 0.08 and 0.145, more preferably equal to 0.11;

wherein LAC is a quantity measured in [mMol/L];
wherein, in step E, said third index (PRR) is calculated during extracorporeal circulation by means of a logistic regression also deriving from said second index.

8. Method according to any one of the preceding claims, wherein step C further comprises receiving a minimum value of hematocrit (HCT) detected during the extracorporeal circulation time ($T_{CPB}$), said detection being an automatic detection,

and wherein the calculation step D further comprises calculating a second index deriving from said minimum value of hematocrit (AKIriskHCT), said second index being calculated by the formula:

- linear regression within a first predetermined range of values of hematocrit (HCT), in particular between 10 and 26 [%]

$$AKIriskHCT = B14 + B15 * HCT$$

wherein:

- B14 is a numerical value comprised between 0.16 and 1.9, preferably comprised between 0.144 and 1.15, more preferably equal to 0.65,
- B15 is a numerical value comprised between -0.082 and 0.014, preferably comprised between -0.042 and 0.001, more preferably equal to -0.02;
- substantially constant within a second predetermined range of values, in particular between 26 and 100 [%], said constant being in particular equal to 0.064;

wherein HCT is a quantity measured in [%];
wherein, in step E, said third index (PRR) is calculated during extracorporeal circulation by means of a logistic regression also deriving from said second index.

9. Method according to any one of the preceding claims, wherein step C further comprises receiving information of transfusions (TRANSF) detected during the extracorporeal circulation time ($T_{CPB}$), said detection being a manual detection,

and wherein the calculation step D further comprises calculating a second index deriving from said information of transfusions (AKIriskTRANSF), said second index being calculated by the formula:

- constant if the patient has not been subjected to transfusion, said constant being in particular equal to 0.082;
- constant if the patient has been subjected to transfusion, said constant being in particular equal to 0.293;

wherein, in step E, said third index (PRR) is calculated during extracorporeal circulation by means of a logistic regression also deriving from said second index.

10. Method according to claims 6 and 7 and 8 and 9, wherein said third index PRR calculated in step E is calculated by the formula:

$$PRR = exp(C1 + C2 * AKIriskT_{CPB} + C3 * AKIriskLAC + C4 * AKIriskDO2 + C5 \\ * AKIriskHCT + C6 * AKIriskT_{DO2} + C7 * AKIriskTRANSF + C8 \\ * AKIriskMAP)/[1 + exp(C1 + C2 * AKIriskT_{CPB} + C3 * AKIriskLAC \\ + C4 * AKIriskDO2 + C5 * AKIriskHCT + C6 * AKIriskT_{DO2} + C7 \\ * AKIriskTRANSF + C8 * AKIriskMAP)]$$

wherein:

- C1 is a numerical value comprised between -6.4 and -1.8, preferably comprised between - 5.83 and -2.39, more preferably equal to -4.11,
- C2 is a numerical value comprised between -0.01 and 9.2, preferably comprised between 1.03 and 8.07, more preferably equal to 4.55,
- C3 is a numerical value comprised between 0.07 and 8.32, preferably comprised between 1.07 and 7.33, more preferably equal to 4.2,
- C4 is a numerical value comprised between -3.7 and 11.3, preferably comprised between - 1.88 and 9.48, more preferably equal to 3.8,
- C5 is a numerical value comprised between -7.3 and 8.5, preferably comprised between -5.3 and 6.46, more preferably equal to 0.58,
- C6 is a numerical value comprised between -9.3 and 9.8, preferably comprised between - 6.94 and 7.5, more preferably equal to 0.28,
- C7 is a numerical value comprised between -1.15 and 8.44, preferably comprised between 0.01 and 7.29, more preferably equal to 3.65,
- C8 is a numerical value comprised between -19.8 and 20.8, preferably comprised between - 0.48 and 1.48, more preferably equal to 0.5.

11. Method according to any one of the preceding claims, wherein said fourth index (MDPI) calculated in step F is calculated by the formula:

$$MDPI = exp(D1 + D2 * SR + D3 * PRR)/[1 + exp(D1 + D2 * SR + D3 * PRR)]$$

wherein:

- D1 is a numerical value comprised between -3.95 and -2.74, preferably comprised between
- 3.8 and -2.9, more preferably equal to -3.35,
- D2 is a numerical value comprised between 0.33 and 8.4, preferably comprised between 1.25 and 7.09, more preferably equal to 4.17,
- D3 is a numerical value comprised between 2.58 and 10.15, preferably comprised between 3.31 and 7.41, more preferably equal to 5.37.

12. Method according to any one of the preceding claims, further comprising the steps of:
B0. displaying on a screen numerically (210) and/or graphically (220) said first index (SR).

13. Method according to any one of the preceding claims, further comprising the steps of:
D0. displaying on a screen a numerical value and/or a time trend of one or more of said at least three second indices ($AKIriskT_{CPB}$, AKIriskDO2, $AKIriskT_{DO2}$).

14. Method according to any one of the preceding claims, further comprising the steps of:
E0. displaying on a screen a numerical value (510) and/or a time trend (520) of said third index (PRR).

15. Method according to any one of the preceding claims, further comprising the steps of:

G0. comparing said fourth index (MDPI) with respect to said first index (SR) and activating signalling means as a function of the difference between said fourth index (MDPI) and said first index (SR);
wherein said signalling means are adapted to provide a graphic signal and/or a sound signal.

EP 4 586 275 A2

100 — Receive settings of first parameters relating to the patient before starting extracorporeal circulation

200 — Calculate a first static index (SR) of the presence of AKI risk deriving from the first parameters

300 — Receive at least three second parameters (TCPB, DO2, TDO2) relating to the patient during extracorporeal circulation

400 — Calculate at least three second dynamic indices (AKIriskTCPB, AKIriskDO2, AKIriskTDO2) of the presence of AKI risk referred to one of the at least three second parameters, respectively

500 — Calculate a third dynamic index (PRR) of the presence of AKI risk referred to all second indices

600 — Calculate a fourth global index (MDPI) of the presence of AKI risk from the first index (SR) and from the third index (PRR)

700 — Display on a screen a numerical value and/or a time trend of said fourth index (MDPI) during extracorporeal circulation

Fig. 1

Fig. 2

Fig. 3

Fig. 4

Fig. 5A

Fig. 5B

Fig. 5C

Fig. 6

Fig. 7